(19) Europäisches Patentamt
European Patent Office
Office européen des brevets

(11) Veröffentlichungsnummer: **0 162 015 B1**

# (12) EUROPÄISCHE PATENTSCHRIFT

(45) Veröffentlichungstag der Patentschrift: **04.12.91**

(51) Int. Cl.$^5$: **C07D 209/08**

(21) Anmeldenummer: **85810220.5**

(22) Anmeldetag: **09.05.85**

## (54) Verfahren zur Herstellung von Indolinen.

(30) Priorität: **16.05.84 US 610727**

(43) Veröffentlichungstag der Anmeldung:
**21.11.85 Patentblatt 85/47**

(45) Bekanntmachung des Hinweises auf die Patenterteilung:
**04.12.91 Patentblatt 91/49**

(84) Benannte Vertragsstaaten:
**AT BE CH DE FR GB IT LI NL SE**

(56) Entgegenhaltungen:
**DE-A- 2 328 330**
**DE-B- 606 027**
**GB-A- 2 051 065**
**JP-A-52 108 969**
**US-A- 4 474 969**

**ACTA CHEMICA SCANDINAVICA, Band 28, Nr. 4, 1974, Seiten 393-398; J. BAKKE et al.: "A new one step indole synthesis"**

**PATENT ABSTRACTS OF JAPAN, Band 8 (C-77), 1977, Seite 4682 C 77; & JP-A-52 142 063**

(73) Patentinhaber: **CIBA-GEIGY AG**
**Klybeckstrasse 141**
**CH-4002 Basel(CH)**

(72) Erfinder: **Rusek, Milos**
**Tiefengrabenstrasse 49**
**CH-4102 Binningen(CH)**
Erfinder: **Kny, Hermann, Dr.**
**Giebenacherstrasse 44**
**CH-4414 Füllinsdorf(CH)**

**EP 0 162 015 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Indolinen der Formel I,

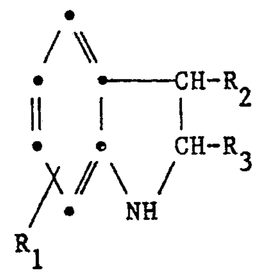

(I)

worin $R_1$ Wasserstoff oder $C_1$-$C_4$-Alkyl, $R_2$ und $R_3$ unabhängig voneinander je Wasserstoff, $C_{1-4}$-Alkyl oder Phenyl bedeutet, durch katalytische Cyclodehydratisierung eines 2-(2'-Aminophenyl)-äthanols der Formel II,

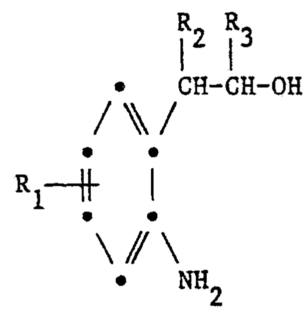

(II)

in welcher $R_1$, $R_2$ und $R_3$ die obige Bedeutung haben, in der Gasphase.

Die Indoline der Formel I sind Zwischenprodukte zur Herstellung von Pyrrolo[3,2,1-i,j]chinolinen mit bakteriziden und fungiziden Eigenschaften, welche zur Bekämpfung von Pflanzenkrankheiten verwendet werden können. Solche Pyrrolo[3,2,1-i,j]chinoline sind in den britischen Patentschriften 1 394 373 und 1 394 374 beschrieben. Als Vertreter dieser Stoffklasse sei insbesondere das 4-Lilolidon (1,2,5,6-Tetrahydro-4H-pyrrolo-[3,2,1-i,j]-chinolin-2-on) der Formel III

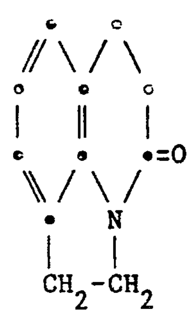

(III)

erwähnt.

Aus der deutschen Patentschrift 606 027 ist bekannt, Indolin in der Weise herzustellen, dass man 2-(2'-Aminophenyl)-äthanol in Gegenwart eines Kondensationsmittels auf 150-500 °C, insbesondere 200-400 °C erhitzt. Als Beispiele für Kondensationsmittel werden die Oxide von Aluminium, Titan, Zirkon, Chrom, Thor oder Gemische dieser Oxide erwähnt. Ferner können nach den Angaben der vorgenannten deutschen Patentschrift auch andere Verbindungen, wie Sulfate, Phosphate, Silikate, Hydrosilikate, Borate, Chloride, Mineralsäuren, und Säureanhydride entweder anstelle der vorgenannten Oxide oder im Gemisch mit ihnen als Kondensationsmittel verwendet werden.

Ferner ist es aus Acta Chem. Scand B 28 (1974) 393-398 bekannt, Indolin durch Ueberleiten von 2-(2'-Aminophenyl)-äthanol-Dampf und Wasserstoff über Silicagel bei 250 °C herzustellen. Es wird eine Ausbeute von 98% berichtet.

Weiterhin ist aus der japanischen Patentanmeldung Sho 52-108.969 bekannt, Indoline durch Erhitzen von entsprechenden 2-(2'-Aminophenyl)-äthylalkoholen auf 200-300 °C herzustellen. Dabei muss der Druck so gewählt werden, dass das Reaktionsgemisch mindestens zum Teil flüssig bleibt.

Schliesslich ist aus der japanischen Patentanmeldung Sho 58-146.562 ein Verfahren bekannt, nach dem Indolin in der Weise hergestellt wird, dass man 2-(2'-Aminophenyl)-äthanol-Dampf bei 180-300 °C über einen Katalysator leitet, der aus mit einer Bor- oder Phosphorverbindung imprägnierten Tonerde oder Kieselgel besteht, wobei als Bor- oder Phosphorverbindung in erster Linie Borsäure und Phosphorsäure in Betracht kommen.

Obwohl nach den vorgenannten Verfahren Indoline in guter Ausbeute erhalten werden können, sind diese Verfahren für eine technische Herstellung von Indolinen wegen der raschen Desaktivierung der verwendeten Katalysatoren und wegen des mit der Regeneration der Katalysatoren verbundenen Aufwandes wenig geeignet.

Es war daher das Ziel der vorliegenden Erfindung, ein Verfahren zur Herstellung von Indolinen der Formel I bereitzustellen, das die Nachteile der bekannten Verfahren vermeidet und das die technische Herstellung der Indoline der Formel I in einfacher Weise und in guter Ausbeute ermöglicht.

Es wurde nun gefunden, dass man die Indoline der Formel I in ausgezeichneter Ausbeute durch Cyclodehydratisierung eines 2-(2'-Aminophenyl)-äthanols der Formel II in der Gasphase herstellen kann, wenn man die Cyclodehydratisierung eines 2-(2'-Aminophenyl)-äthanols der Formel II bei 200-350 °C in Gegenwart eines Silikats oder eines Alumosilikats eines Erdalkalimetalls oder eines seltenen Erdmetalls und in Gegenwart von Trägergas durchführt.

Der zur Durchführung der Reaktion bevorzugte Temperaturbereich ist 240-260 °C. Wegen der Endothermie der erfindungsgemässen Cyclodehydratisierung muss Wärme zur Aufrechterhaltung der Reaktionstemperatur dem System zugeführt werden.

Die Cyclodehydratisierung kann erfindungsgemäss unter atmosphärischem oder vermindertem Druck durchgeführt werden. Vorzugsweise wird unter atmosphärischem Druck gearbeitet.

Die Cyclodehydratisierung wird erfindungsgemäss in Gegenwart von Trägergas durchgeführt. Die Verwendung von Wasserstoff, Stickstoff, Kohlensäure oder Wasserdampf als Trägergas ist möglich. Der als Trägergas bevorzugte Wasserdampf kann im Verhältnis von 0,2 bis 10 Mol pro Mol 2-(2'-Aminophenyl)-äthanol eingesetzt werden. Besonders bevorzugt ist der Einsatz von 3 bis 5 Mol Wasserdampf pro Mol 2-(2'-Aminophenyl)-äthanol. Die Zugabe von Wasser kann über getrennte Wasser- oder Dampfleitungen erfolgen oder dadurch, dass die entsprechend konzentrierte wässrige Lösung des 2-(2'-Aminophenyl)-äthanols über einen Verdampfer in den Reaktor geführt wird.

Die zur erfindungsgemässen Cyclodehydratisierung verwendbaren Erdalkalisilikat-Katalysatoren können durch Umsetzung eines in Wasser gelösten Alkaliwasserglases, wie z.B. Kaliwasserglas mit der wässrigen Lösung eines Erdalkalimetallsalzes, wie z.B. Magnesiumnitrat, durch Filtrieren, Waschen und Trocknen des resultierenden und ausgeschiedenen Erdalkalisilikats und durch dessen Aktivierung durch Luftdurchleitung bei Temperaturen von 300 bis 550 °C hergestellt werden.

Der bevorzugte Temperaturbereich für die Aktivierung der Erdalkalisilikatkatalysatoren beträgt 340-360 °C.

Von den zur erfindungsgemässen Umsetzung erwähnten Erdalkalisilikat-Katalysatorenwird das Magnesiumsilikat bevorzugt verwendet.

Als Katalysatoren für die erfindungsgemässe Cyclodehydratisierung eignen sich die der Gruppe der Silikate oder Alumosilikate zugehörigen synthetischen oder natürlichen Molekularsiebe. Solche Molekularsiebe mit Kationen von Erdalkali- oder seltenen Erdmetallen können aus den Alkalimetall-Kationen enthaltenden synthetischen Molekularsieben durch Ionenaustausch hergestellt werden, wie das in Firmenschriften beschrieben ist. (Z.B.: "Linde Molecular Sieves, Catalyst Bulletin, Union Carbide", 270 Park Ave., New York, N.Y. 10017, USA).

Molekularsiebe, welche vorwiegend Kationen von seltenen Erdmetallen enthalten, werden als Katalysatoren ebenfalls bevorzugt verwendet. Solche Katalysatoren sind käuflich.

Der unter der Bezeichnung Strem 14-8910 angebotene Katalysator ist beispielsweise ein mit seltenen Erden angereichertes Alumo-Silikat Molekularsieb mit folgender Zusammensetzung:

| | |
|---|---|
| $SiO_2$ | 65,0 % |
| $Al_2O_3$ | 22,7 % |
| $Na_2O$ | 1,6 % |
| $Re_2O_3$ | 10,7 %, |

wobei Re die Summe aller Kationen von seltenen Erdmetallen bedeutet.

Zur katalytischen Cyclodehydratisierung können Reaktoren mit bewegtem Bett, z.B. ein Wirbelschichtreaktor, oder Festbettreaktoren eingesetzt werden. Die Aktivierung oder Reaktivierung des Katalysators kann in einem der erwähnten Reaktoren erfolgen, parallel zur Reaktion oder abwechslungsweise damit.

Das erfindungsgemässe Verfahren ist für eine kontinuierliche Prozeßführung geeignet.

Das erfindungsgemässe Verfahren ist allgemein anwendbar für die Herstellung der Indoline der Formel I, worin $R_1$ Wasserstoff oder $C_1$-$C_4$-Alkyl, $R_2$ und $R_3$ unabhängig voneinander je Wasserstoff, $C_{1-4}$-Alkyl oder Phenyl bedeuten. Unter $C_1$-$C_4$-Alkyl werden die Reste Methyl, Aethyl, n-Propyl, Isopropyl, primär und sekundär n-Butyl, Isobutyl und tert.-Butyl umfasst. Das Verfahren ist für die Herstellung des Indolins ($R_1$, $R_2$, $R_3$ = H) bevorzugt anwendbar.

Nach einer insbesondere bevorzugten Ausführungsform des erfindungsgemässen Verfahrens wird das 2-(2'-Aminophenyl)-äthanol in Gegenwart von Mg-Silikat und unter Zusatz von 3-5 Mol Wasserdampf pro Mol 2-(2'-Aminophenyl)-äthanol bei 240-260° zu Indolin cyclodehydratisiert.

Die zur erfindungsgemässen Cyclodehydratisierung eingesetzten substituierten 2-(2'-Aminophenyl)-äthanole können durch Reduktion der entsprechenden Nitroverbindungen hergestellt werden. Das 2-(2'-Aminophenyl)-äthanol ist auch aus dem 2-(2'-Nitrophenyl)-äthanol durch Reduktion mit Zink nach Sabetay (Bull. Soc. Chim. Fr. 1931, 49 3) leicht erhältlich.

Das 2-(2'-Nitrophenyl)-äthanol und seine substituierten Derivate sind durch Addition von o-Nitroalkylbenzolen an einen Aldehyd nach der japanischen Patentschrift 77-108941; C.A. Vol. 88 (1978) 104875x nach folgendem Reaktionsschema herstellbar:

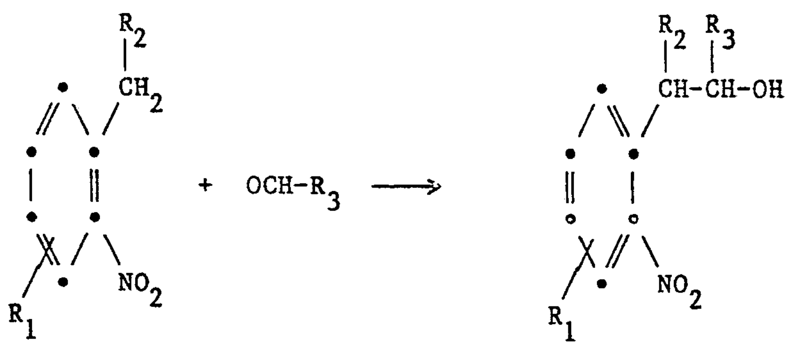

Mit dem erfindungsgemässen Verfahren können Indoline durch katalytische Cyclodehydratisierung von 2-(2'-Aminophenyl)-äthanolen mit einer Selektivität von über 97% bei einem Umsatz von mindestens 98% hergestellt werden, wobei vorteilhafterweise die Lebensdauer des Katalysators im Vergleich zu den bisher bekannten Verfahren vielfach verlängert wird. Dieser vorteilhafte Effekt wird erfindungsgemäss durch Trägergaszusatz erzielt.

Obwohl ein Mol Wasser pro Mol eingesetztes 2-(2'-Aminophenyl)-äthanol entsteht, ist der erfindungsgemässe Wasserzusatz für den Reaktionsverlauf wegen der Aktivierung des Katalysators vorteilhaft. Dieser Effekt ist als überraschend zu bezeichnen.

Das erfindungsgemässe Verfahren wird durch folgende Beispiele näher erläutert:

Beispiel 1:

400 g Magensiumnitrat, Hexahydrat, gelöst in 6 L Wasser wird mit 720 ml Kaliumwasserglas (9,6% Si, d = 1,262 bei 20°) gelöst in 6 L Wasser verrührt. Der gebildete Niederschlag wird abfiltriert, gewaschen, getrockent und bei 350° 5 Stunden lang gehalten.

Vom so hergestellten Katalysator werden 3 ml in das Katalysatorbett eines Mikroreaktors eingesetzt. 3 ml/Stunde einer 65%-igen wässrigen Lösung von 2-(2'-Aminophenyl)-äthanol werden bei atmosphärischem Druck in den Reaktor dosiert. Die Temperatur des Katalysators wird bei 260° C gehalten.

Das aus dem Reaktor austretende Gasgemisch wird gaschromatographisch analysiert. Während eines Versuchs von der Dauer von vier Tagen werden konstant folgende Resultate registriert: Umsatz von 2-(2'-Aminophenyl)-äthanol: 98,1-99,9% des Einsatzes Selektivität zum Indolin 95,3-98,3 % bezüglich Umsatz.

Beispiel 2:

In einem Rohrreaktor werden 100 ml Molekularsieb "10x" (Union Carbide) als Katalysator eingesetzt. Der Reaktor wird mit einer Geschwindigkeit von 100 ° C/Std. auf 250 ° geheizt.

68,5 g (0,5 M) 2-(2'-Aminophenyl)-äthanol/Stunde wird als 65%-ige wässrige Lösung in den Reaktor dosiert. Als zusätzliches Trägergas wird 9 L/Stunde Stickstoff, verunreinigt mit 5% Kohlenwasserstoffen zugeführt. Die Temperatur wird während der 24 stündigen Reaktion bei 250 ° C gehalten. Die Umsetzung findet unter atmosphärischem Druck statt.

Die Resultate betragen durchschnittlich

Umsatz von 2-(2'-Aminophenyl)-äthanol: 99% des Einsatzes Selektivität zum Indolin 97 Mol-% des umgesetzten Alkohols.

Beispiel 3:

In einen Mikroreaktor werden 2,45 ml Molekularsieb (Strem Chem. 14-8910, enthält 10,7% Oxyde von seltenen Erdmetallen) als Katalysator eingesetzt. Der Reaktor wird mit einer Geschwindigkeit von 100 ° C/Std. auf 250 ° geheizt.

3 ml/Stunde einer 65% wässrigen Lösung von 2-(2'-Aminophenyl)-äthanol wird bei atmosphärischem Druck in den Reaktor dosiert; die Temperatur wird bei 240 ° C gehalten.

Die Resultate betragen während 8 Stunden durchschnittlich: Umsatz von 2-(2'-Aminophenyl)-äthanol: 99% des Einsatzes, Selektivität zum Indolin 98 Mol-%.

**Patentansprüche**

**1.** Verfahren zur Herstellung von Indolinen der Formel I,

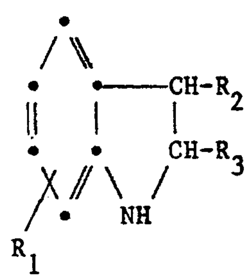

(I)

worin $R_1$ Wasserstoff oder $C_1$-$C_4$-Alkyl, $R_2$ und $R_3$ unabhängig voneinander je Wasserstoff, $C_{1-4}$-Alkyl oder Phenyl bedeutet, durch katalytische Cyclodehydratisierung eines 2-(2'-Aminophenyl)-äthanols der Formel II,

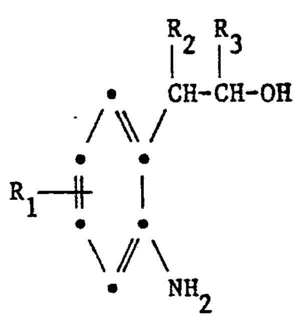

(II)

in welcher $R_1$, $R_2$ und $R_3$ die obige Bedeutung haben, in der Gasphase bei 200-350 °, dadurch

gekennzeichnet, dass man die Cyclodehydratisierung in Gegenwart eines Silikats oder eines Alumosilikat eines Erdalkalimetalls oder seltenen Erdmetalls, als Katalysator und von Trägergas durchführt.

2. Verfahren nach Anspruch 1, dadurch gekennzeichnet, dass man die Reaktion bei 240-260°C durchführt.

3. Verfahren nach Anspruch 1, dadurch gekennzeichnet, dass man die Reaktion unter atmosphärischem Druck durchführt.

4. Verfahren nach Anspruch 1, dadurch gekennzeichnet, dass man als Trägergas 0,2 bis 10 Mol Wasserdampf pro Mol 2-(2'-Aminophenyl)-äthanol der Reaktion zusetzt.

5. Verfahren nach Anspruch 4, dadurch gekennzeichnet, dass man als Trägergas 3 bis 5 Mol Wasserdampf pro Mol 2-(2'-Aminophenyl)-äthanol der der Reaktion zusetzt.

6. Verfahren nach Anspruch 1, dadurch gekennzeichnet, dass man die Reaktion kontinuierlich durchführt.

7. Verfahren nach Anspruch 1, dadurch gekennzeichnet, dass man den Katalysator periodisch durch Durchblasen von Luft und Stickstoff bei 300-550° aktiviert.

8. Verfahren nach Anspruch 1, dadurch gekennzeichnet, dass der Katalysator ein Molekularsieb ist, an dessen Oberfläche Kationen von seltenen Erdmetallen oder Erdalkalimetallen gebunden sind.

9. Verfahren nach Anspruch 1, dadurch gekennzeichnet, dass man das 2-(2'-Aminophenyl)-äthanol in Gegenwart von Mg-Silikat und unter Zusatz von 3-5 Mol Wasserdampf pro Mol 2-(2'-Aminophenyl)-äthanol bei 240-260° zu Indolin cyclodehydratisiert.

**Claims**

1. A process for the preparation of an indoline of the formula I

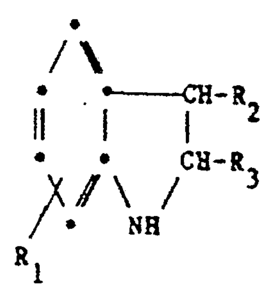

(I)

wherein $R_1$ is hydrogen or $C_1$-$C_4$alkyl, and $R_2$ and $R_3$ are each independently hydrogen, $C_1$-$C_4$alkyl or phenyl, by catalytic cyclodehydration of a 2-(2'-aminophenyl)ethanol of the formula II

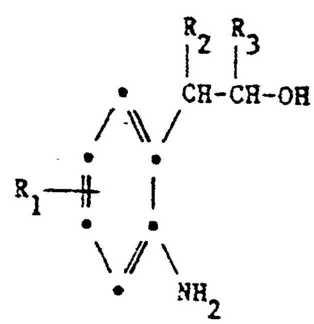

(II)

wherein $R_1$, $R_2$ and $R_3$ are as defined above, in the gaseous phase at 200°-350°, which process

comprises carrying out the cyclodehydration in the presence of a silicate or an aluminosilicate of an alkaline earth metal or rare earth metal as catalyst, and in the presence of a carrier gas.

**2.** A process according to claim 1, wherein the reaction is carried out in the temperature range from 240°-260°C.

**3.** A process according to claim 1, wherein the reaction is carried out under atmospheric pressure.

**4.** A process according to claim 1, wherein 0.2 to 10 moles of water vapour per mole of 2-(2'-aminophenyl)-ethanol are added to the reaction as carrier gas.

**5.** A process according to claim 4, wherein 3 to 5 moles of water vapour per mole of 2-(2'-aminophenyl)-ethanol are added to the reaction as carrier gas.

**6.** A process according to claim 1, wherein the reaction is carried out continuously.

**7.** A process according to claim 1, wherein the catalyst is periodically activated by blowing air and nitrogen through in the temperature range from 300°-550°.

**8.** A process according to claim 1, wherein the catalyst is a molecular sieve to the surface of which are attached cations of rare earth metals or alkaline earth metals.

**9.** A process according to claim 1, wherein the 2-(2'-aminophenyl)-ethanol is cyclodehydrated to indoline at 240°-260° in the presence of magnesium silicate and with the addition of 3 to 5 moles of water vapour per mole of 2-(2'-aminophenyl) ethanol.

## Revendications

**1.** Procédé de préparation d'indolines de formule I

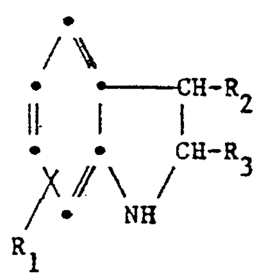

(I)

dans laquelle $R_1$ représente l'hydrogène ou un groupe alkyle en C1-C4, $R_2$ et $R_3$ représentent chacun, indépendamment l'un de l'autre, l'hydrogène, un groupe alkyle en C1-C4 ou phényle, par cyclodéshydratation catalytique d'un 2-(2'-aminophényl)-éthanol de formule II

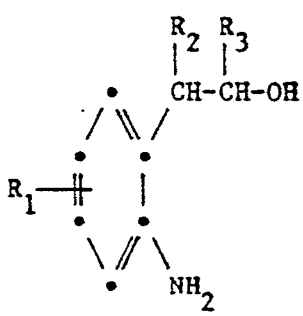

(II)

dans laquelle $R_1$, $R_2$ et $R_3$ ont les significations indiquées ci-dessus, en phase gazeuse, à des

températures de 200 à 350° C, caractérisé en ce que l'on effectue la cyclodéshydratation en présence d'un silicate ou d'un aluminosilicate d'un métal alcalino-terreux ou d'un métal des terres rares qui sert de catalyseur, et d'un gaz véhicule.

**2.** Procédé selon la revendication 1, caractérisé en ce que l'on effectue la réaction à des températures de 240 à 260° C.

**3.** Procédé selon la revendication 1, caractérisé en ce que l'on effectue la réaction à pression atmosphérique.

**4.** Procédé selon la revendication 1, caractérisé en ce que l'on introduit dans la réaction, en tant que gaz véhicule, de la vapeur d'eau en quantité de 0,2 à 10 mol par mol du 2-(2'-aminophényl)-éthanol.

**5.** Procédé selon la revendication 4, caractérisé en ce que l'on introduit dans la réaction, en tant que gaz véhicule, de la vapeur d'eau en quantité de 3 a 5 mol par mol du 2-(2'-aminophényl)-éthanol.

**6.** Procédé selon la revendication 1, caractérisé en ce que l'on effectue la réaction en continu.

**7.** Procédé selon la revendication 1, caractérisé en ce que l'on active périodiquement le catalyseur par injection d'air et d'azote à des températures de 300 à 550°.

**8.** Procédé selon la revendication 1, caractérisé en ce que le catalyseur est un tamis moléculaire portant, fixé sur sa surface, des cations de métaux des terres rares ou de métaux alcalino-terreux.

**9.** Procédé selon la revendication 1, caractérisé en ce que l'on convertit le 2-(2'-aminophényl)-éthanol en indoline par cyclodéshydratation en présence de silicate de magnésium et avec adjonction de 3 à 5 mol de vapeur d'eau par mol du 2-(2'-aminophényl)-éthanol à des températures de 240 à 260° C.